# EUROPEAN PATENT SPECIFICATION

(11) **EP 2 753 311 B1**
(45) Date of publication and mention of the grant of the patent: **29.07.2020**
(21) Application number: 12829455.0
(22) Date of filing: 10.09.2012
(51) Int. Cl.: A61P 39/00, A61P 39/06, A61K 36/82, A61K 36/53, A61K 36/534, A61K 36/537, A23K 20/174, A23K 20/105, A23K 20/111, A23K 20/158, A23K 20/10, A23K 50/40

(54) **ANTIOXIDANT FORMULATIONS**
ANTIOXIDATIVE FORMULIERUNGEN
FORMULATIONS D'ANTIOXYDANTS

(30) Priority: 09.09.2011 US 201161532859 P
(43) Date of publication of application: 16.07.2014
(73) Proprietor: Kemin Industries, Inc., Des Moines, Iowa 50317 (US)
(72) Inventor: CUTLER, Sara, Carlisle Iowa 50047 (US); SZAJNA-FULLER, Ewa, Ames, Iowa 50010 (US); ROTBERG, Isabella, Boynton Beach Florida 33472 (US); WRAY, Carrie, Pleasant Hill Iowa 50327 (US); TRUONG, My, Des Moines Iowa 50316 (US); POSS, Mitchell, Johnston Iowa 50131 (US)
(74) Representative: Evans, Jacqueline Gail Victoria
(86) International application number: PCT/US2012/054466
(87) International publication number: WO 2013/036934

(56) References cited:
- WO-A1-01/26472
- WO-A1-2009/105626
- WO-A2-2010/096597
- FR-A1- 2 899 768
- US-A1- 2006 141 073
- US-A1- 2007 059 344
- US-A1- 2008 085 338
- US-A1- 2011 152 371
- US-A1- 2012 046 369
- US-A1- 2012 225 050
- NANJO F ET AL: "Effects of dietary tea catechins on [alpha]-tocopherol levels, lipid peroxidation, and erythrocyte deformability in rats fed on high palm oil and perilla oil diets", BIOLOGICAL & PHARMACEUTICAL BULLETIN (OF JAPAN), PHARMACEUTICAL SOCIETY OF JAPAN, TOKYO, JP, vol. 16, no. 11, 1 November 1993 (1993-11-01), pages 1156-1159, XP009182557, ISSN: 0918-6158
- ZHU Q Y ET AL: "REGENERATION OF ALPHA-TOCOPHEROL IN HUMAN LOW-DENSITY LIPOPROTEIN BY GREEN TEA CATECHIN", JOURNAL OF AGRICULTURAL AND FOOD CHEMISTRY, AMERICAN CHEMICAL SOCIETY, US, vol. 47, no. 5, 1 May 1999 (1999-05-01), pages 2020-2025, XP000824115, ISSN: 0021-8561, DOI: 10.1021/JF9809941
- RYOKO HASHIMOTO ET AL: "Inhibition of Radical Reaction of Apolipoprotein B-100 and [alpha]-Tocopherol in Human Plasma by Green Tea Catechins", JOURNAL OF AGRICULTURAL AND FOOD CHEMISTRY, vol. 48, no. 12, 1 December 2000 (2000-12-01), pages 6380-6383, XP55169258, US ISSN: 0021-8561, DOI: 10.1021/jf000973i
- RAU O ET AL: "Carnosic acid and carnosol, phenolic diterpene compounds of the Labiatae herbs rosemary and sage, are activators of the human peroxidsome proliferator-activated receptor gamma", MEDICINAL & AROMATIC PLANTS ABSTRACTS, SCIENTIFIC PUBLISHERS, SCIENTIFIC PUBLISHERS, NEW DELHI - INDIA, vol. 28, no. 6, 1 December 2006 (2006-12-01), XP018019891, ISSN: 0250-4367
- TERNES W ET AL: "ANTIOXIDATIVE CONSTITUENTS OF ROSMARINUS OFFICINALIS AND SALVIA OFFICINALIS IV. DETERMINATION OF CARNOSIC ACID IN DIFFERENT FOODSTUFFS", ZEITSCHRIFT FUER LEBENSMITTELUNTERSUCHUNG UND -FORSCHUNG, XX, XX, vol. 201, no. 6, 1 January 1995 (1995-01-01), pages 548-550, XP000893141, ISSN: 0044-3026, DOI: 10.1007/BF01201582
- TANG S Z ET AL: "Effects of added tea catechins on colour stability and lipid oxidation in minced beef patties held under aerobic and modified atmospheric packaging conditions", JOURNAL OF FOOD ENGINEERING, BARKING, ESSEX, GB, vol. 77, no. 2, 1 November 2006 (2006-11-01), pages 248-253, XP027891076, ISSN: 0260-8774 [retrieved on 2006-11-01]

## Description

### Background of the Invention

This application claims priority to United States Patent Application Serial No. 61/532,859, filed September 9, 2011.

The present invention relates generally to antioxidant formulations for pet food containing lipid-soluble tea catechins and tocopherol.

Antioxidants are applied at several stages of the pet food kibble manufacturing process before, during, and after extrusion. One common antioxidant comprises mixed tocopherols and/or tocotrienols. In one particular example, Naturox® Plus Dry (Kemin Industries, Inc., Des Moines, Iowa) is a dry antioxidant (DA) mixture which is added to the kibble dry recipe before it is extruded, while Naturox® Premium Liquid (Kemin Industries, Inc., Des Moines, Iowa) is a liquid antioxidant (LA) formulation in oil added to the enrobing fat on the kibble's surface. Since LA is applied closest to the air/kibble interface, it is crucial to the oxidative stability of the kibble. The LA formulation may also be applied to the meat meal during its production in the rendering process or directly after the rendering when the meal is isolated from the offal, to control oxidation of protein and fat prior to be used in the dry meal.

Green and black teas, as well as other varieties of tea, are well known to have water soluble antioxidants which perform well in a hydrophilic food matrix. Previous attempts at suspending these water soluble antioxidants in oil with AAFCO (Association of American Feed Control Officials) approved ingredients at concentrations needed for use in antioxidant formulations have been unsuccessful.

### Summary of the Invention

Recently, lipid soluble catechins (LSC) were identified to have antioxidant properties and maintain solubility in hydrophobic media, including vegetable oils. Oxidative Stability Index (OSI) results of LSC in animal and vegetable fat were promising and, as a result, the material was used in this sequence of trials. These materials, in conjunction with rosemary extract, natural surfactants (lecithin) and chelators (citric acid), were paired in formulas with the goal of creating a more versatile and equal, if not improved, formula with better efficacy.

New dry antioxidant formulations were developed to include not only fat soluble antioxidants, chelators, surfactants, but water soluble antioxidant extracts from spearmint and rosemary, which provided for an improved level of efficacy, especially at accelerated temperature storage conditions. As part of the antioxidant dry formulations, the chelators were varied and included organic acids, inorganic phosphate, milk whey protein and polyphosphate chelators with a targeted range in pH from 1.5 to 12. Surfactants for the dry formulations included lecithin, but also may include other ionic or non-ionic surfactants that are naturally derived or from non-natural sources, to either solubilize, act as a synergist and enhancing the antioxidant properties, or to further distribute the antioxidant into the desired host matrix.

For all formulations mixed tocopherols includes a single iso-form or a mixture of all structural isomers (alpha, beta, gamma, etc.) of tocopherols and/or tocotrienols.

The present invention provides an antioxidant composition for use in enhancing the stability of animal feed and constituents of animal feed comprising a tocopherol and lipid soluble tea catechins.

### Brief Description of the Drawings

Fig. 1 is a chart of peroxide values (meq/kg diet) of kibbles treated with 1000 ppm of 8 different liquid antioxidant formulations stored at 65 °C
Fig. 2 is a comparison between OSI and PV (time to rancidity) results in sunflower oil treated with antioxidants

### Description of the Invention

The best antioxidant choice for stabilization of any given product depends on multiple complex factors. One of the factors that affects the selection is the polarity of the antioxidant. The polarity of the antioxidant affects where the antioxidant is located in the product and whether it can interact with free radicals. For example, polar antioxidants are effective in bulk oil situations due to what is referred to as the polar paradox. The polar paradox states that polar antioxidants do not like a non-polar oil matrix and will concentrate at polar interfaces. In a bulk oil these interfaces include air/oil interfaces and water/oil interfaces often in the form of emulsified micelles. A non-polar antioxidant is not as effective as the antioxidant is simply diluted and dispersed in the general oil matrix and not concentrated at the interface. The opposite has been observed with animal protein meals and pet food diets, where non-polar antioxidants have been observed to perform the best. Numerous trials have shown that the polar antioxidants do not perform as well in these meal and diet matrices.

Previous work has attempted to utilize the top antioxidants that performed well in the AOCS Official Method Cd 12b-92 oil stability index (OSI) to identify the best antioxidants for stabilization. As expected in an OSI test which measures stability on bulk oils, the polar antioxidants perform the best due to the polar paradox described earlier. It is common practice in the industry to use the OSI as an antioxidant screening tool. It has been our observations that the OSI is not an appropriate tool for predicting the best antioxidants for meals or diets, and goes counter to accepted practice. Top performing polar antioxidants include examples such as water soluble green tea, gallic acid, ascorbic acid, etc. In particular, water soluble green tea extracts contain a substantial quantity of the unmodified or natural leaf polyphenols many of which are synthesized into the natural form of catechins (see, e.g., US 2007/0286932). These antioxidants perform very poorly when they are used for meal and diet stabilization. While the best antioxidants for meals and diets are non-polar, there are a limited number of natural non-polar or oil soluble antioxidants. Examples include tocopherols, tocotrienols, carnosic acid, etc. There are other non-polar antioxidants but they do not have favorable commercial pricing. The present invention discloses for the first time the use of lipid soluble catechins which have the advantage of being oil soluble, economically viable and suitable for replacing a large amount of conventional antioxidants while still providing effective stabilization of meals and diets. The lipid soluble catechins are shown to provide meal and diet stabilization beyond what is achievable in water soluble forms of catechins.

In the present invention, the antioxidants, individually or in combination, can be added to the overall diet or to the oil used in the diet.

Tocopherols are traditionally applied to diets in amounts between 50 and 250 ppm. Tocopherols are known to be prooxidants in oils above about 5000 ppm. The ranges of tocopherols applied to oils and diets in the present invention are between 10 ppm and about 250 ppm with a preferred range or between 40 ppm and 240 ppm.

In the present invention, rosemary extracts are used in the range of between 0 and 100 ppm to the diet, with a preferred range of between 0 ppm and 60 ppm to the diet, and between 0 ppm and 360 ppm to the oil with a preferred range of between 0 ppm and 200 ppm to the oil.

In the present invention, lipid soluble catechins are used in the range of between 0 and 120 ppm to the diet, with a preferred range of between 10 ppm and 60 ppm to the diet, and between 10 ppm and 150 ppm to the oil with a preferred range of between 10 ppm and 75 ppm to the oil.

### Example 1 - Addition of Lipid Soluble Tea Extract

### Materials and Methods

Liquid antioxidant formulas, the compositions of which are listed Table 1, were applied to extruded kibble in enrobing fat.

**Table 1. Active ingredients of liquid antioxidant formulations.**

| **Treatment Name** | **Tocopherols (%)** | **Rosemary (%)** | **LSC (%)** |
|---|---|---|---|
| LA 1 | 0 | 0 | 0 |
| LA 2 | 24 | 0.1 | 0 |
| LA 3 | 20 | 0.1 | 2 |
| LA 4 | 17 | 0.1 | 5 |

The chicken fat was treated with 3000 ppm of the liquid antioxidant formulas prior application to the kibble at 4.5%. Palatant was also applied to the kibble at 1%. Finished kibble was stored at 47 °C in individual plastic bags and analyzed for peroxide values (PV) using the FOX II Method (Gülgün Yildiz, Randy L. Wehling and Susan L. Cuppett. Comparison of four analytical methods for the determination of peroxide value in oxidized soybean oils. Journal of the American Oil Chemists' Society Volume 80, Number 2, 2003, 103-107; Nourooz-Zadeh, Jaffar; Tahaddine-Sarmadi, Javad; Birlouez-Aragon, Ines; and Wolff, Simon P. Measurement of Hydroperoxides in Edible Oils Using the Ferrous Oxidation in Xylenol Orange Assay. J. Agric Food Chem, Bol 43. No. 1. 1995, 17-21) every 2 weeks. Formation of hexanal and 2,4-decadienal was measured at week 4 by gas chromatography (Frankel, EN. Methods to determine extent of oxidation. In: Lipid Oxidation. The Oily Press: Dundee, Scotland. Copyright 1998). The results are presented in Table 2.

**Table 2. Peroxide values and aldehydes (sum of hexanal and 2,4-decadienal) levels of the kibble stored at 47 °C for 4 weeks.**

| **Treatment Name** | **PV (mEq/kg sample)** | **Aldehydes (ppm)** |
|---|---|---|
| LA 1 | 4.2 | 156 |
| LA 2 | 2.1 | 69 |
| LA 3 | 1.4 | 48 |
| LA 4 | 1.3 | 43 |

After 4 weeks of storing the kibble at 47 °C the study was terminated as the peroxide values for all of the treatments reached 1 mEq/kg, which is considered an indication of rancidity. As expected, lack of antioxidants (LA1) resulted in highest peroxide value as well as level of aldehydes. More importantly, formulas containing LSC outperformed tocopherol-based antioxidant, as apparent from the peroxides values (1.4, 1.3 for LA3, LA4 vs. 2.1 for LA2) and aldehydes content (48, 43 for LA3, LA4 vs. 69 for LA2).

### Example 2 - Addition of Spearmint Extract

Dry antioxidant formulations, listed in Table 3, were added to kibble dry mix with a ribbon blender and extruded in sequence. Water soluble green tea extract (WSGT) standardized to 45% epigallocatechin gallate and 45% other catechins was obtained from Kemin Industries, Inc. (Des Moines, Iowa).

**Table 3. Active ingredients of dry antioxidant formulations.**

| **Treatment Name** | **Tocopherols (%)** | **Rosemary (%)** | **WSGT (%)** | **LSC (%)** | **Spearmint (%)** |
|---|---|---|---|---|---|
| DA 1 | 0 | 0 | 0 | 0 | 0 |
| DA 2 | 22 | 0.1 | 0 | 0 | 0 |
| DA 3 | 11 | 5 | 6 | 0 | 0 |
| DA 4 | 11 | 5 | 0 | 6 | 0 |
| DA 5 | 11 | 5 | 0 | 0 | 5 |

The kibbles were coated with untreated chicken fat at 4.5% and palatant at 1%, and placed in storage at 25 °C, 37 °C and 47 °C. Samples were analyzed for peroxide values (PV) using the FOX II Method and secondary lipid oxidation products (hexanal and 2,4 decadienal) by gas chromatography. Results are shown in Table 4.

**Table 4. Peroxide values and aldehydes (sum of hexanal and 2,4-decadienal) levels of the kibble stored at ambient temperature, 37 °C and 47 °C.**

| **Treatment Name** | **PV (mEq/kg sample)** | **Aldehydes (ppm)** |
|---|---|---|
| | Ambient (16 weeks) | |
| DA 1 | 1.9 | 57 |
| DA 2 | 1.0 | 28 |
| DA 3 | 1.6 | 44 |
| DA 4 | 0.7 | 21 |
| DA 5 | 0.7 | 18 |
| | 37 °C (12 weeks) | |
| DA 1 | 7.0 | 296 |
| DA 2 | 5.6 | 186 |
| DA 3 | 5.8 | 234 |
| DA 4 | 2.8 | 77 |
| DA 5 | 1.5 | 38 |
| | 47 °C (4 weeks) | |
| DA 1 | 4.2 | 156 |
| DA 2 | 6.3 | 184 |
| DA 3 | 5.3 | 187 |
| DA 4 | 1.1 | 29 |
| DA 5 | 1.1 | 26 |

The inclusion of dry antioxidant into the kibble results in higher oxidative stability as evident from lower peroxide values and aldehyde levels under the storage conditions. Antioxidant formulas containing LSC and spearmint extract performed substantially better than the tocopherol-based formula, especially at higher temperatures. Interestingly, the LSC and WSGT containing formulations demonstrated vast differences in performance, showing that the water-soluble green tea extract did not control oxidation in the pet food matrix tested.

### Example 3 - Evaluation of Antioxidant Activity of Lipid Soluble Tea Catechins (LSC) by OSI

The effectiveness of LSC extract in combination with tocopherols, rosemary extract, and lecithin was tested using an animal fat as a matrix. Formulations listed in Table 5 were applied to the fat at 1000 ppm and 3000 ppm levels.

**Table 5. Composition of formulas**

| **Treatment Name** | **Tocopherols (%)** | **Rosemary (%)** | **LSC (%)** |
|---|---|---|---|
| 0% LSC | 22 | 0.1 | 0 |
| 1% LSC | 21 | 0.1 | 1 |
| 2% LSC | 20 | 0.1 | 2 |
| 3% LSC | 19 | 0.1 | 3 |
| 5% LSC | 17 | 0.1 | 5 |

The induction period of the fat treated with antioxidant formulations (Table 6) was compared to the untreated fat.

**Table 6. OSI results for chicken fat treated with antioxidant formulas.**

| | **OSI (hr)** | |
|---|---|---|
| **Treatment Name** | **1000 ppm** | **3000 ppm** |
| Untreated | 5.9 | |
| 0% LSC | 21.6 | 31.1 |
| 1% LSC | 22.1 | 31.9 |
| 2% LSC | 23.7 | 35.8 |
| 3% LSC | 24.1 | 37.7 |
| 5% LSC | 25.4 | 42.9 |

OSI results show that the antioxidant activity of the formulas increased with higher LSC content. Samples containing 5% LSC applied to the fat at 3000 ppm had the highest induction period among tested formulations.

### Example 4 - Evaluation of Antioxidant Efficacy at High Temperatures

Fat samples were treated with 1000 and 3000 ppm of experimental antioxidant formulas having varying ratios of tocopherols, rosemary extract, lipid soluble tea catechins (LSC) and lecithin, as shown in Table 7, and tested in duplicate in the OSI at 100 ºC (Table 8).

**Table 7. LA Prototypes tested in the LSC storage study.**

| **Treatment Name** | **Tocopherols (%)** | **Rosemary (%)** | **LSC (%)** | **Lecithin (%)** |
|---|---|---|---|---|
| LSC-1 | 0 | 0 | 0 | 0 |
| LSC-2 | 24 | 0.1 | 0 | 0 |
| LSC-3 | 12 | 6 | 3 | 2 |
| LSC-4 | 18 | 0 | 4 | 2 |
| LSC-5 | 15 | 0 | 7 | 2 |
| LSC-6 | 12 | 0 | 10 | 2 |
| LSC-7 | 0 | 12 | 12 | 2 |
| LSC-8 | 0 | 5 | 18 | 2 |

**Table 8. OSI results of LSC formulas in chicken fat.**

| | **OSI (h)** | |
|---|---|---|
| **Treatment Name** | **1000 ppm** | **3000 ppm** |
| LSC-1 | 6.7 | 6.7 |
| LSC-2 | 30.7 | 56.7 |
| LSC-3 | 35.7 | 62.5 |
| LSC-4 | 35.7 | 54.0 |
| LSC-5 | 39.1 | 57.1 |
| LSC-6 | 16.4 | 34.4 |
| LSC-7 | 14.0 | 30.6 |
| LSC-8 | 34.6 | 50.7 |

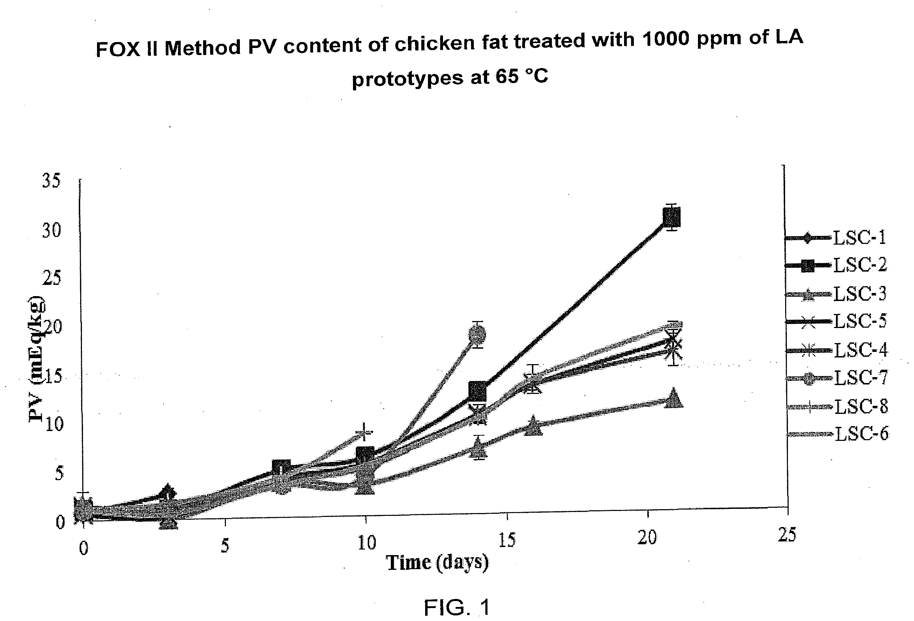

Additionally, 9 g treated poultry fat was weighed into an OSI tube, stored in an OSI unit at 65 °C and connected to air flow tubing. The progress of oxidation was measured by analyzing the rise in peroxide values over time (Figure 1).

The performance of the liquid formulations containing LSC was equivalent or improved when tested in the OSI at 65 °C. Formula LSC-3 out-performed all other formulas at 65 °C, and was statistically equivalent in the OSI to the current Naturox® Premium Liquid.

### Example 5 - Synergy Between Antioxidants

Experiments were conducted to study the effect of combination of antioxidants on the time to rancidity of sunflower oil. Sunflower oil was treated with tocopherol at 1200 ppm alone and combined with WSGT (350 ppm), rosemary extract (250 ppm) and LSC (350 ppm) and placed in an incubator at 40 ºC. Samples of the sunflower oil were periodically analyzed for peroxide values (PV) using the FOX II Method. Time to rancidity (PV≥10 meq/kg oil) was determined for all of the treatments. Results show the increase in stability of sunflower oil treated with combinations of antioxidants (Table 9) in contrast to the treatment with tocopherols alone.

**Table 9. Synergistic Effect of Antioxidant Combinations on Time to Rancidity**

| | **Time to rancidity (days)** | **Stability increase** |
|---|---|---|
| Tocopherol (1200 ppm) | 9 | |
| Tocopherol (1200 ppm) + WSGT (350 ppm) | 14 | 56% |
| Tocopherol (1200 ppm) + Rosemary (250 ppm) | 21 | 133% |
| Tocopherol (1200 ppm) + LSC (350 ppm) | 28 | 211% |

Tocopherols are known to be especially effective in stabilizing sunflower oil. However, a marked and unexpected increase in stability was observed with the addition of lipid soluble catechins. This increase is significantly longer than what was observed with the water soluble green tea (WSGT) and is counter to what was observed by the OSI results.

### Example 6 - Comparison of Stability of Sunflower Oil in OSI and PV Score

According to the American Oil Chemist Society, the Oil Stability Index (OSI) is the point of maximum change in an oil of fat's oxidation under standard conditions. Accordingly, the OSI determines the relative resistance of an oil or fat to oxidation and is an indicator of the length of shelf life for that fat or oil. Experiments were done to evaluate the effect of lipid soluble catechins on the OSI of sunflower oil and on the shelf life of sunflower oil.

Sunflower oil was treated with four different antioxidants: tocopherol at 1200 ppm (total tocopherol concentration); rosemary at 250 ppm (Rosan™ SF 35 from Kemin Industries, Inc., a rosemary extract standardized to 10% carnosic acid); water soluble green tea extract at 35 ppm (standardized to 45% EGCG and 45% other catechins); lipid soluble catechins at 35 ppm (standardized to 74% catechins). Untreated sunflower oil was used at the control. A shelf life study of the same samples at ambient temperature was also conducted. Shelf life time to rancidity was defined as the number of days before the peroxide value (PV) exceeded 10 meq/kg. The results are set out in Table 10.

**Table 10. Time to Rancidity of Sunflower Oil**

| **Name** | **OSI (h)** | **Time to rancidity (days)** |
|---|---|---|
| Untreated | 11.45 | 9 |
| Tocopherol (1200 ppm) | 14.95 | 9 |
| Rosemary (250 ppm) | 26.35 | 21 |
| WSGT (350 ppm) | 31.65 | 14 |
| LSC (350 ppm) | 19.15 | 35 |

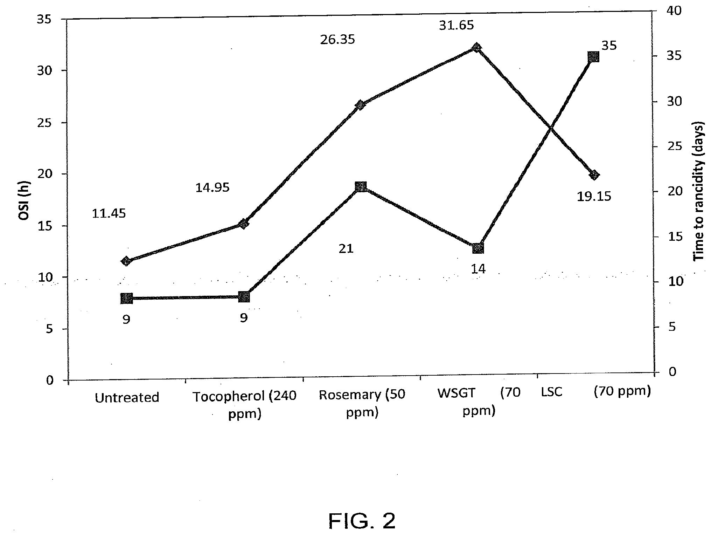

The results show that, surprisingly, the OSI results were not predictive of shelf life for lipid soluble catechins (Fig. 2). The lipid soluble catechins are much more effective at extending shelf life than was expected from the OSI results.

### Example 7 - Synergy Between Antioxidants

Experiments were conducted to study the effect of antioxidants alone and in combinations on the peroxide value and 2,4-decadienal values of kibble after 6 weeks at 37 °C. In a first set of experiments, the poultry fat used to coat the kibble was either left untreated or treated with 240 ppm tocopherol, 50 ppm rosemary, 70 ppm WSGT, or 70 ppm LSC. The results are shown in Table 11. In a second set of experiments, the fat used to coat the kibble was either left untreated or treated with 240 ppm tocopherol plus 50 ppm rosemary extract, 240 ppm tocopherol plus 70 ppm WSGT, , 240 ppm tocopherol plus 70 ppm LSC, 50 ppm rosemary plus 70 ppm WSGT, , and 50 ppm rosemary plus 70 ppm LSC. The results are shown in Table 12.

**Table 11 - Effect of Antioxidant Combinations on Peroxide and 2,4-Decadienal Values**

| **Treatment Name** | **PV (mEq/kg sample)** | **2,4-Decadienal (mEq/kg sample)** |
|---|---|---|
| Untreated fat | 19.2 | 22 |
| 240 ppm tocopherol | 14.7 | 18 |
| 50 ppm rosemary | 17.7 | 20 |
| 70 ppm WSGT | 16.7 | 18 |
| 70 ppm WSGT base | 17.7 | 20 |
| 70 ppm LSC | 21.6 | 24 |

**Table 12 - Effect of Antioxidant Combinations on Peroxide and 2,4-Decadienal Values**

| **Treatment Name** | **PV (mEq/kg sample)** | **2,4-Decadienal (mEq/kg sample)** |
|---|---|---|
| Untreated fat | 19.2 | 22 |
| 240 ppm tocopherol + 50 ppm rosemary | 14.7 | 17 |
| 240 ppm tocopherol + 70 ppm WSGT | 14.9 | 17 |
| 240 ppm tocopherol + 70 ppm WSGT base | 15.7 | 19 |
| 240 ppm tocopherol + 70 ppm LSC | 15.0 | 17 |
| 50 ppm tocopherol + 50 ppm WSGT | 20.8 | 24 |
| 50 ppm tocopherol + 50 ppm WSGT base | 17.1 | 19 |
| 50 ppm tocopherol + 70 ppm LSC | 16.0 | 18 |

From Table 11 it is seen that the lipid soluble catechins when used alone did not perform as well as the other antioxidants and indeed did not perform as well as leaving the fat untreated. WSGT was one of the better performing antioxidants, which again matched with the observations from the OSI testing. However, when used in combination with tocopherol (Tables 9 and 12), the lipid soluble catechins provided a synergistic protective effect, enabling a reduction in tocopherol to approximately one-fifth of the prior inclusion level without significantly increasing either the peroxide or 1,4-decadienal values. A key goal of the pet food industry has been to reduce the use of tocopherols in the formulations. It has previously been difficult to reduce tocopherol concentrations due to difficulty finding synergistic antioxidants that are effective in a combination that matches the stabilization capability of tocopherols on products under real world storage conditions. In this work we've been able to reduce tocopherol levels up to 80% and still provide similar or better shelf life on a finished pet food diet. This work has shown synergism between tocopherols and LSC in combination or in addition to other antioxidants.

The foregoing description and drawings comprise illustrative embodiments of the present inventions.

## Claims

1. An antioxidant composition for enhancing the stability of animal feed and constituents of animal feed comprising a tocopherol and lipid soluble tea catechins.

2. A composition as defined in claim 1 further comprising a carnosic acid-containing extract of a Lamiaceae spp. plant.

3. A composition as defined in claim 2, wherein said Lamiaceae spp. plant is selected from the group consisting of basil, mint, rosemary, sage, savory, marjoram, oregano, thyme and lavender.

4. A composition as defined in claim 1 further comprising a rosmarinic acid -containing extract of a Lamiaceae spp. plant.

5. A method of improving the stability of an animal food composition or a constituent of an animal food composition comprising the step of adding an antioxidant composition comprising a tocopherol and lipid soluble tea catechins to said food composition or constituent.

6. A method as defined in claim5 , wherein the antioxidant composition further comprises a carnosic acid-containing extract of a Lamiaceae spp. plant.

7. A method as defined in claim 6, wherein said Lamiaceae spp. plant is selected from the group consisting of basil, mint, rosemary, sage, savory, marjoram, oregano, thyme and lavender.

8. A method according to claim 5 wherein the antioxidant composition further comprises a rosmarinic acid -containing extract of a Lamiaceae spp. plant.

9. A method for protecting animal fat for use as a pet food or pet food constituent from oxidation during rendering, comprising adding an antioxidant composition according to any one of claims 1 to 4 to the fat prior to the rendering process or during the rendering process.

## Patentansprüche

1. Antioxidanszusammensetzung zur Verbesserung der Stabilität von Tierfutter und Bestandteilen von Tierfutter, umfassend ein Tocopherol und lipidlösliche Teekatechine.

2. Zusammensetzung nach Anspruch 1, ferner umfassend einen Carnosinsäure enthaltenden Extrakt einer Lamiaceae spp.-Pflanze.

3. Zusammensetzung nach Anspruch 2, wobei die Lamiaceae spp.-Pflanze ausgewählt ist aus der Gruppe bestehend aus Basilikum, Minze, Rosmarin, Salbei, Bohnenkraut, Majoran, Oregano, Thymian und Lavendel.

4. Zusammensetzung nach Anspruch 1, ferner umfassend einen Rosmarinsäure enthaltenden Extrakt einer Lamiaceae spp.-Pflanze.

5. Verfahren zur Verbesserung der Stabilität einer Tierfutterzusammensetzung oder eines Bestandteils einer Tierfutterzusammensetzung, umfassend den Schritt des Hinzufügens einer Antioxidanszusammensetzung, die ein Tocopherol und lipidlösliche Teekatechine umfasst, zu der Futterzusammensetzung oder dem Futterbestandteil.

6. Verfahren nach Anspruch 5, wobei die Antioxidanszusammensetzung ferner einen Carnosinsäure enthaltenden Extrakt einer Lamiaceae spp.-Pflanze umfasst.

7. Verfahren nach Anspruch 6, wobei die Lamiaceae spp.-Pflanze ausgewählt ist aus der Gruppe bestehend aus Basilikum, Minze, Rosmarin, Salbei, Bohnenkraut, Majoran, Oregano, Thymian und Lavendel.

8. Verfahren nach Anspruch 5, wobei die Antioxidanszusammensetzung ferner einen Rosmarinsäure enthaltenden Extrakt einer Lamiaceae spp.-Pflanze umfasst.

9. Verfahren zum Schutz von tierischem Fett zur Verwendung als Haustierfutter oder Haustierfutterbestandteil vor Oxidation während der Verwertung, umfassend das Hinzufügen einer Antioxidanszusammensetzung nach einem der Ansprüche 1 bis 4 zu dem Fett vor dem Verwertungsprozess oder während des Verwertungsprozesses.

## Revendications

1. Composition d'antioxydants destinée à accroître la stabilité d'aliments pour animaux et de constituants d'aliments pour animaux comprenant un tocophérol et des catéchines de thé solubles dans les lipides.

2. Composition telle que définie dans la revendication 1 comprenant en outre un extrait contenant de l'acide carnosique d'une plante Lamiaceae spp.

3. Composition telle que définie dans la revendication 2, dans laquelle ladite plante Lamiaceae spp. est choisie parmi le groupe constitué du basilique, de la menthe, du romarin, de la sauge, de la sarriette, de la marjolaine, de l'origan, du thym et de la lavande.

4. Composition telle que définie dans la revendication 1 comprenant en outre un extrait contenant de l'acide rosmarinique d'une plante Lamiaceae spp.

5. Procédé d'amélioration de la stabilité d'une composition de nourriture pour animaux ou d'un constituant d'une composition de nourriture pour animaux comprenant l'étape d'ajout d'une composition d'antioxydants comprenant un tocophérol et des catéchines de thé solubles dans les lipides à ladite composition de nourriture ou audit constituant.

6. Procédé tel que défini dans la revendication 5, dans lequel la composition d'antioxydants comprend en outre un extrait contenant de l'acide carnosique d'une plante Lamiaceae spp.

7. Procédé tel que défini dans la revendication 6, dans lequel ladite plante Lamiaceae spp. est choisie parmi le groupe constitué du basilique, de la menthe, du romarin, de la sauge, de la sarriette, de la marjolaine, de l'origan, du thym et de la lavande.

8. Procédé selon la revendication 5, dans lequel la composition d'antioxydants comprend en outre un extrait contenant de l'acide rosmarinique d'une plante Lamiaceae spp.

9. Procédé destiné à protéger de la graisse animale destinée à être utilisée comme une nourriture pour animaux domestiques ou un constituant de nourriture pour animaux domestiques contre l'oxydation pendant la récupération, comprenant l'ajout d'une composition d'antioxydants selon l'une quelconque des revendications 1 à 4 à la graisse avant le processus de récupération ou pendant le processus de récupération.
